# EUROPEAN PATENT APPLICATION

(11) **EP 1 785 148 A2**
(43) Date of publication of application: **16.05.2007**
(21) Application number: 06023562.9
(22) Date of filing: 13.11.2006
(51) Int. Cl.: A61L 2/18, A61L 2/26, A61B 1/12

(54) **Apparatus for washing and disinfecting medical instruments**

(30) Priority: 11.11.2005 JP 2005327889
(71) Applicant: OLYMPUS MEDICAL SYSTEMS CORP., Shibuya-ku Tokyo 151-0072 (JP)
(72) Inventor: Noguchi, Toshiaki, Tachikawa-shi Tokyo 190-0002 (JP); Suzuki, Eiri, Sagamihara-shi Kanagawa-ken 229-0038 (JP)
(74) Representative: von Hellfeld, Axel

(57) **Abstract**

An apparatus (2) is provided for washing and disinfecting a medical instrument (20) with a duct (22a). The apparatus comprises an accommodating member (10) on which the medical instrument is accommodated and a bath member (3) providing a washing bath (5) in which the accommodating member is placed. The apparatus further comprises a washing unit (16c, 17c, 26, 41-77) washing at least the duct of the medical instrument accommodated on the accommodating member and placed in the washing bath and a brushing unit (85) detachably mounted on the bath member and formed to have a brush (27a) brushing an inside of the duct when the endoscope is washed and disinfected by the washing unit.

## Description

### CROSS REFERENCE TO RELATED APPLICATION

The patent application related to and incorporates by reference Japanese Patent application No. 2005-327889 filed on Nov. 11, 2005.

### Background of the Invention

### (The field of the Invention)

The present invention relates to an apparatus for washing and disinfecting used medical instruments, such as endoscopes.

### (Related Art)

Medical endoscopes have now been used widely for examining and treating bodily ills. In order to cope with such applications, a medical endoscope has a thin and elongated tube (simply, referred to as an insertion tube) inserted into body cavities of an object being examined and treated. This insertion tube provides one or more ducts formed therethrough. The ducts include endoscopic ducts (channels), such as a suction channel (duct) serving as a channel for various biopsy forceps.

When such an insertion tube is inserted into body cavities, the outer surface of an insertion tube and the insides of endoscopic channels thereof are polluted with humor and filth. Hence it is required to sufficiently wash and disinfect the outer surface and inside of the endoscopic channels of an endoscope whenever this has been used.

By way of example, Japanese Patent Laid-open Publication No. 11-76145 discloses a washing device for washing an endoscope. This endoscope has a brush inserting tube to guide a long washing brush into an endoscopic channel (duct) for washing.

However, in the case of this washing device, the long washing brush should be inserted through the brush inserting tube and the brush should be handled by hand so as to repeatedly advance and retreat through an endoscopic channel. This washing is very troublesome and time-consuming work to operators. In addition, for exchanging the washing brushes, the long washing brush to be disposed of, which has been used so far, is normally rolled up or folded to make it compact. However, this rolling or folding operation also imposes troublesome labor on operators.

### Summary of the Invention

The present invention has been made in consideration of the foregoing difficulties and has an object to provide an apparatus for washing and disinfecting ducts of a medical instrument that has been used, in particular, endoscopic channels (ducts) of an endoscope that has been used, with reliability and with effectiveness in the washing and disinfecting work.

As one aspect, the present invention provides an apparatus (2) for washing and disinfecting a medical instrument (20) with a duct (22a). The apparatus comprises an accommodating (mounting) member (10) on which the medical instrument is accommodated (mounted) and a bath member (3) providing a washing bath (5) in which the accommodating member is placed. The apparatus further comprises a washing unit (16c, 17c, 26, 41-77) washing at least the duct of the medical instrument accommodated on the accommodating member and placed in the washing bath and a brushing unit (85) detachably mounted on the bath member and formed to have a brush (27a) brushing an inside of the duct when the endoscope is washed and disinfected by the washing unit.

Accordingly, a used medical instrument, such as endoscope, is simply accommodated on the accommodating member and the apparatus is switched on, whereby the medical instrument is washed by the washing unit. The duct of the medical instrument (for example, the suction channel of an endoscope) is brushed by the brush of the brushing unit. In consequence, the medical instrument can be washed in a sanitary and less-labor manner. Especially, manual troublesome brushing work is not required.

As another aspect, the present invention provides a detachable type of brushing apparatus (85), which comprises a detachable member (88, 88a) detachably mounted to a detachable portion (84a) of a washing and disinfecting apparatus (2) for washing and disinfecting a medical instrument (20) with a duct (22a) and formed to provide an approach path (38) to the duct of the medical instrument; a flow path (86, 88b, 90) communicating with the approach path; and a brushing unit (27, 87a, 87b, 87c) disposed in the flow path and formed to have a brush (27a) advancing an inside of the duct through the approach path and brushing the inside of the duct, when the duct is subjected to at least washing. Preferably, the apparatus further comprises a tank (95a, 95b) containing at least one of washing fluid and disinfecting fluid necessary for washing and disinfection of the duct and a guiding path (95c) guiding the fluid contained in the tank to the flow path.

Accordingly, it is enough to the detachable brushing apparatus is mounted on the washing and disinfecting apparatus, which allows the brush to be inserted into the duct of the medical instrument for brushing therein. This brushing apparatus may have a tank for containing the washing and/or disinfecting fluid. In such a structure, there is no necessity to install the tank in the washing and disinfecting apparatus, simplifying the handing of the tank. Further, the washing and disinfecting apparatus is simplified in terms of its piping and control mechanism. The detachable brushing apparatus may be either disposed of when being used up or re-used.

### Brief Description of the Drawings

In the accompanying drawings:
Fig. 1 is a perspective view outlining the overall configuration of an endoscope washing and disinfecting apparatus serving as a medical instrument washing and disinfecting apparatus according to a first embodiment of the present invention;
Fig. 2 is a perspective view showing the endoscope washing and disinfecting apparatus whose washing bath is loaded with an endoscope and whose top cover is closed;
Fig. 3 is a schematic view showing the piping configuration in the endoscope washing and disinfecting apparatus;
Fig. 4 is a plan view, partially sectioned, of a nozzle-mounted unit of the apparatus;
Fig. 5 is a perspective view showing the appearance of a washing brush unit detachably mounted on the nozzle-mounted unit;
Fig. 6 is a plan view showing the washing brush unit;
Fig. 7 is a longitudinal cross sectional view of the washing brush unit;
Fig. 8 is a plan view showing a mounting surface of the nozzle-mounted unit, the mounting surface being subjected to detachable mounting of the wash brushing unit;
Fig. 9 is a sectional view showing a positional relationship between the washing brush unit and the muzzle-mounted device embedded in the apparatus and exposed to the outside from the apparatus;
Fig. 10 is a plan view showing the washing brush unit mounted on the mounting surface, that is, the nozzle-mounted unit;
Fig. 11 is a plan view showing a state where a used endoscope is accommodated in the endoscope washing and disinfecting apparatus;
Figs. 12 and 13 are illustrations each showing a motion of the nozzle-mounted unit mounted on the washing brush unit;
Fig. 14 is a perspective view showing a washing brush unit adopted by a second embodiment of the endoscope washing and disinfecting apparatus according to the present invention;
Fig. 15 is a plan view of the washing brush unit adopted in the second embodiment;
Fig. 16 is a longitudinal sectional view of the washing brush unit mounted on the nozzle-mounted unit in the second embodiment;
Fig. 17 is an illustration explaining an inside of the washing brush unit in the second embodiment;
Figs. 18 and 19 explain how the fluid is supplied from tanks in the washing brush unit according to the second embodiment; and
Figs. 20 and 21 show schematic views each showing the piping configuration applicable to the endoscope washing and disinfecting apparatus according to the second embodiment.

### Detailed Description of the Preferred Embodiments

Various embodiments of the present invention will now be described with reference to the accompanying drawings.

### (First Embodiment)

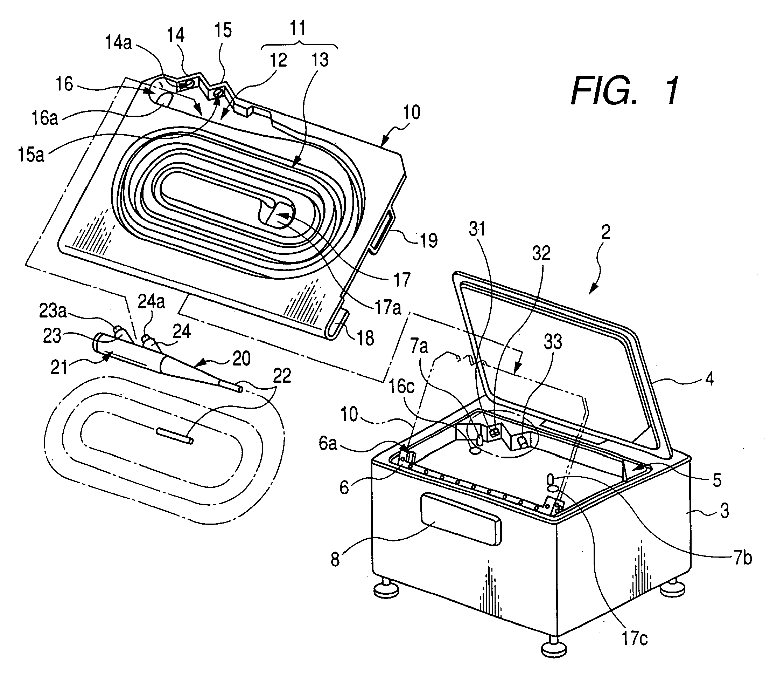

Referring to Figs. 1-13, a first embodiment will now be described. In the present embodiment, the medical washing and disinfecting apparatus is reduced into practice as an endoscope washing and disinfecting apparatus.

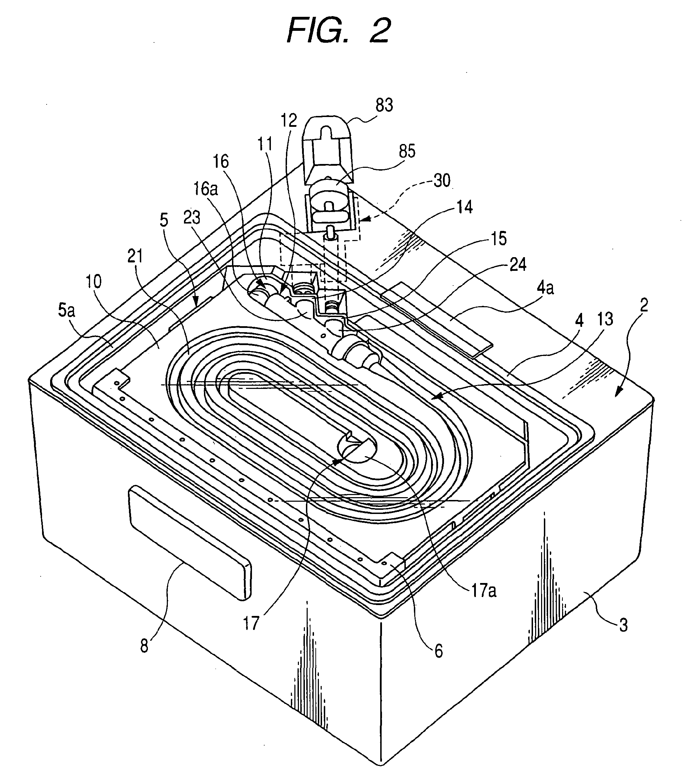

Figs. 1 and 2 illustrate the overall configuration of an endoscope washing and disinfecting apparatus 2, which serves as the medical instrument washing and disinfecting apparatus, according to the present invention.

This endoscope washing and disinfecting apparatus 2 is provided with a body housing 3 in which mechanically and electrically necessary components are incorporated, a top cover 4, and a tray 10. The body housing 3 provides a washing bath 5 of a predetermined depth at the top of the body housing 3. The top cover 4 is disposed to cover the opening of the washing bath 5.

A tray holder 6 is rotatably disposed at an edge of the washing bath 5. A tray 10 for holding used endoscopes, which provides an endoscope loading plane, is detachably disposed at the tray holder 6. The washing bath 5 has a bottom, on which there are disposed first and second protrusions 7a and 7b.

A water supply port 16c is formed in the vicinity of the first protrusion 7a, while a drain port 17c is formed in the vicinity of the second protrusion 7b. Washing fluid and rinsing water is supplied from the water supply port 16c to both the washing bath 5 and the tray 10. The fluid fed from the water supply port 16c is discharged outside the washing bath 5 via the drain port 17c.

On the front of the body housing 3, there is formed an operation panel 8 with which an operator is able to give various data to the apparatus 2 and receive various information from the apparatus 2 in an interactive manner.

The top cover 4, made of hard and light-transmittance resin material, that is, transparent or semitransparent resin material, is formed into a predetermined shape and disposed at an edge of the washing bath 5 to allow the top cover 4 to be open and closed. Hence, even if the top cover 4 is closed to cover the opening of the washing bath 5, an operator can visually observe the inside of the washing bath 5 through the top cover 5.

Incidentally, the endoscope washing and disinfecting apparatus 2 according to the present embodiment is able to cope with washing and disinfecting, besides the endoscopes, other medical tools, such as therapeutic instruments with ducts or tubular through-holes and over tubes. For washing and disinfecting those medical tools, trays dedicated to hold such tools are prepared.

In the present embodiment, an endoscope 20 is mounted on the tray 10 and accommodated or mounted in the washing bath 5. This tray 10 is produced as a holding plate dedicated to this endoscope 20 itself. If the type of an endoscope being washed differs from that of this endoscope 20, another tray produced dedicatededly to the endoscope is used.

As shown in Fig. 1, the present endoscope washing and disinfecting apparatus 2 further comprises washing nozzles 31, 32 and 33 on a side wall near a corner of the washing bath 5. These washing nozzles 31, 32 and 33 are members which can be connected with or disconnected from various channels (ducts) of an endoscope 20 for washing and disinfecting them and compose one of main parts of a nozzle-mounted unit 30 arranged in the body housing 3, as will be described later.

The nozzle-mounted unit 30, which is installed in the body housing 3, has a later-described mounting surface exposed to the outside at a corner of the upper surface of the body housing 3 and a washing brush unit 85 is detachably mounted on the mounting surface, i.e., to the nozzle-mounted unit 30. On this mounting surface, a cover 83 which covers the washing brush unit 95 is provided to be opened and closed (refer to Fig. 2).

The tray 10 is used to mount a used endoscope 20 thereon. The tray holder 6 has a holding member 6a to hold the tray 10, as shown by a chain double-dashed line in Fig. 1.

The endoscope 20 is provided with a base portion 21 and an elongated long insertion tube 22 extended from the base portion 21. This insertion tube 22 is flexible. The base portion 21 comprises an air/water-supply channel connector 23 and a suction channel connector 24, which are obliquely protruded from a side of the base portion 21 to form an acute angle to the base end of the base portion 21. Both connectors 23 and 24 are parallel to each other.

Of these connectors 23 and 24, the air/water-supply channel connector 23 has air/water-supply connecting ports 23a, which are connected to both an air-supply connecting member, to which one end of an air-supply duct is connected, and a water-supply connecting member, to which one end of a water-supply duct is connected, respectively. Meanwhile the suction channel connector 24 has a suction connecting port 24a, which is connected to a suction connecting member, to which one end of a suction channel (duct) 22a is connected. Thus, during an examination with the endoscope 20, the air/water-supply connecting ports 23a are connected with air/water-supply tubes (not shown) to supply air and water to the endoscope 20. Meanwhile, the suction connecting port 24a is connected with a suction tube 22a arranged through the insertion tube 22.

The base portion 21 is used as a gripping portion which is gripped by a user who desires to operate the endoscope 20. In the case of fixedly securing the endoscope 20 to fixing members such as arms, this base portion 21 is used as a fixing member.
On the tray 10 is formed a accommodating groove 11 (a kind of recess) which serves as a guide groove to allow the endoscope 20 to be located and accommodated at predetermined positions on the tray 10. The accommodating groove 11 has a predetermined contour formed in accordance with the outer shapes of both the base portion 21 and the insertion tube 22. This means that the tray 10 is produced to be dedicated to each endoscope 20, type by type, of which accommodating groove 11 is fit to the outer shape of each endoscope 20. If there are plural types of endoscopes in a medical facility (i.e., such endoscopes have base portions and/or insertion tubes having different outer shapes and/or lengths), a plurality of types of trays 10 dedicated to those various type endoscopes may be prepared.

To be specific, the accommodating groove 11 consists of a base-accommodating recess (groove) 12 into which the base 12 of the endoscope 20 is placed for accommodation and an almost vortical tube-accommodating groove 13 into which the insertion tube 22 thereof is placed for accommodation. The tube-accommodating groove 13 is formed into a substantially vortical shape on the tray 10. The recess 12 and the groove are formed as a continuous groove for guiding the placement of the endoscope 20 onto the tray 10.

The base-accommodating recess 12 is provided with, as part of the side wall thereof, an air/water-supply channel accepting member 14 and a suction channel accepting member 15 at both of which the air/water-supply channel connector 23 and the suction channel connector 24 are disposed, respectively. Of these members 14 and 15, the air/water-supply channel accepting member 14 has an opening 14a through which the air/water-supply connecting ports 23a is secured. On the other hand, the suction channel accepting member 15 has an opening 15a through which the suction connecting port 24a is secured.

At a predetermined position of the base of the base-accommodating recess 12, a first water port 16 is formed to feed and discharge water such as washing water and disinfecting water. Similarly, at another predetermined position of the base of the tube-accommodating groove 13, a second water port 17 is formed to feed and discharge water such as washing water and disinfecting water. The first water port 16 is positioned in proximity to the base side of the base portion 21. The second water port 17 is positioned in proximity with a distal surface of the insertion tube 21. Each of the first and second water ports 16 and 17 has a lid 16a (17a) which can be opened and closed. The lids 16a and 17a are held to always close the water ports 16 and 17 by not-shown forcing members combined with their weight. Only the weight of each lid 16a (17a) can be utilized as such forcing means. With no tray placed in the washing bath 5, the lids 16a and 17a are made to close. Thus, when an endoscope 20, which has been used so far, is accommodated in the accommodating groove 11, humor and/or filth on the endoscope 20 is prevented from leaking out through the first and second ports 16 and 17. Therefore, with the endoscope 20 accommodated in the accommodating groove 11 of the tray 10, the endoscope 20 can be carried sanitarily.

In the present embodiment, the first water port 16 is used to feed washing water, disinfecting water, and so on into the accommodating groove 11 therethrough. And, through the second water port 17, the water such as washing water and disinfecting water, which has been fed in the accommodating groove 11, is drained from the groove 11 to the washing base 5. In order to allow the base portion 21 and insertion tube 22 of an endoscope 20 accommodated in the accommodating groove 11 to be immersed sufficiently in the washing of disinfecting water supplied through the first water port 16, the accommodating groove 11 is formed so that there is no useless space left between the endoscope 20 and the wall of the accommodating groove 11. It is therefore possible to minimize amounts of washing water and disinfecting water required.

As shown in Fig. 1, the tray 10 has an attachment portion 18 which is used to attach the tray 10 to the holding member 16a at the longitudinal edge of the washing bath 5. The attachment portion 18 is shaped into for example a U-shaped form, which is adaptable to the holding member 6a. A reference 19 in Fig. 1 shows one of gripping hands for carry. The gripping hands 19 are located on both lateral side edges of the tray 10. To avoid interference with the top cover 4, the gripping hands 19 are made to protrude downward from the tray 10.

When an endoscope 20 is accommodated in the accommodating groove 11 on the tray 10, both connecting ports 23a and 24a of the air/water-supply channel connector 23 and suction channel connector 24 of the endoscope 20 are fixedly placed through the opening 14a of the air/water-supply channel accepting member 14 and the opening 15a of the suction channel accepting member 15, respectively. By this placement, the used endoscope 20 can easily be accommodated in the accommodating groove 11 with a predetermined attitude thereof.

After accommodating the endoscope 20 in the accommodating groove 11, the tray 10 is made to be connected with the tray holder 6, as shown in Fig. 2. A proper attachment of the attachment portion 18 of the tray 10 with the holding member 6a of the tray holder 6 makes it possible that the tray 10 is rotated in the downward by hand or with an automatic mechanism (not shown). Thus the tray 10 can be put into the washing bath 5.

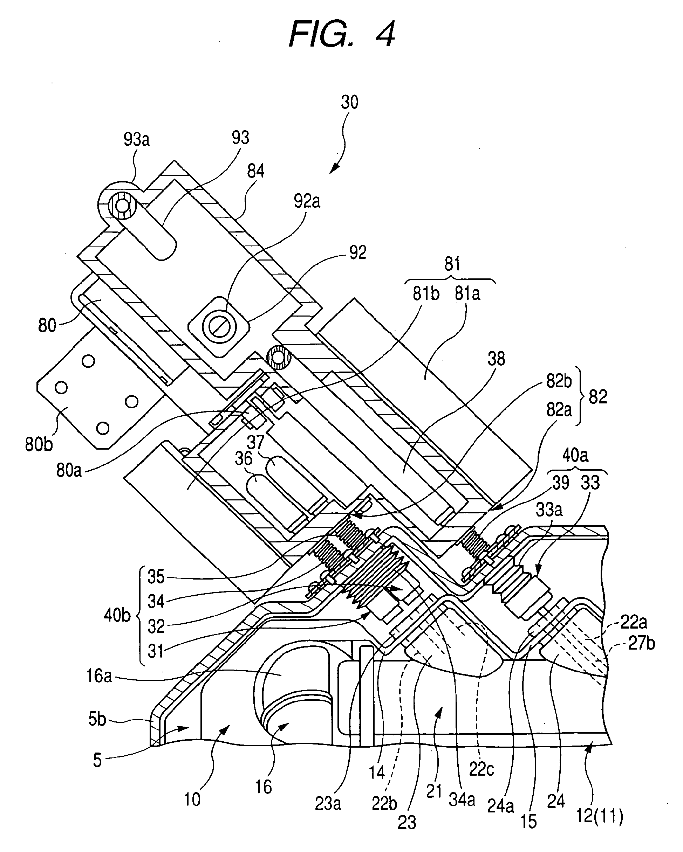

In response to the downward rotation of the tray 10, the first and second protrusions 7a and 7b on the base of the washing bath 5 push up the lids 16a and 17a so as to make the first and second water ports 16 and 17 open, respectively. Concurrently with this open actions of the lids 16a and 17a, as shown in Fig. 4, both connecting ports 23a and 24a of the endoscope 20, which are placed to protrude from the openings 14a and 15b of the side wall of the accommodating groove 11, are located to face an air-supply channel washing nozzle 31, a water-supply channel washing nozzle 32, and a suction channel washing nozzle 33, respectively, with a predetermined distance apart from the nozzles.

When the accommodation of the endoscope 20 is completed, the top cover 4 is moved by hand or with the use of an automatic mechanism (not shown), whereby the washing bath 5 is covered as shown in Fig. 2.

A packing 5a is attached on the upper surface of the body housing 3 in such a manner that the packing 5a surrounds the edge of the washing bath 5. Hence when the top cover 4 closes the washing bath 5, the packing 5a is pressed down by the top cover 4 so as to sustain the watertight performance between the top cover 4 and the washing bath 5. This watertight performance prevents the liquid within the washing bath 5 from scattering outside the body housing 3. A hinge 4a is secured on an edge of the top cover 4 for opening and closing the top cover 4.

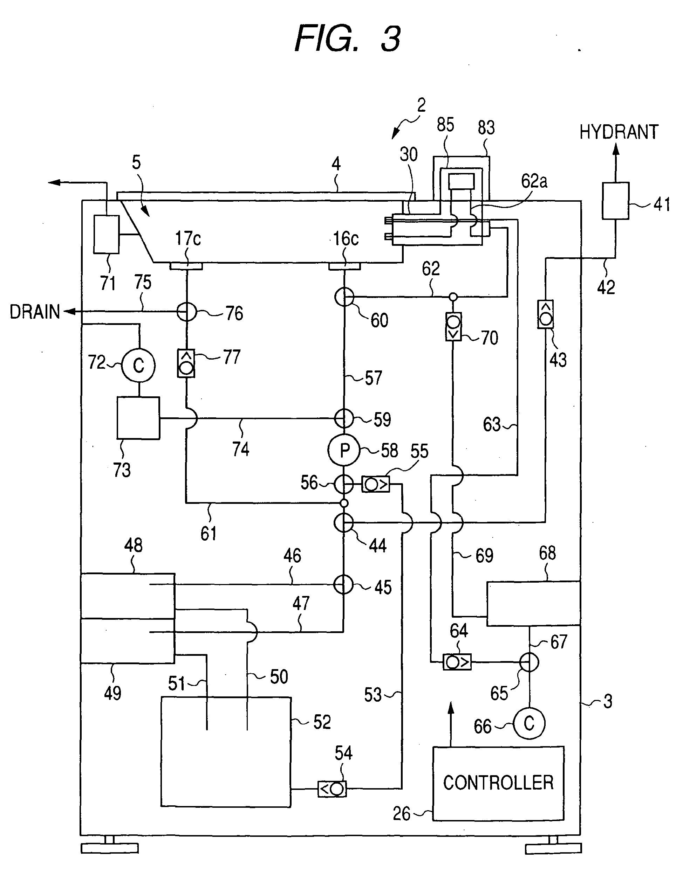

Referring to Fig. 3, the piping structure of the endoscope washing and disinfecting apparatus 2 will now be described.

As illustrated in Fig. 3, the body housing 3 of this apparatus 2 introduces a duct 42 connected to a hydrant, so that the tap water comes into the unit 3 through the duct 42. Along this duct 42, a water filter 42, a check valve 43, and tow three-way switching valves 44 and 45 intervenes in this order from the hydrant side. In addition, this duct 42 is branched into two branch ducts 46t and 47 at the one three-way switching valve 45.

Of these two branch ducts 46 and 47, one duct 46 is linked with a washing agent bottle 48, while the remaining duct 47 is linked with a chemical bottle 49. A duct 50 connects the washing agent bottle 48 and a stirring bath 52 and another duct 51 connects the chemical bottle 49 and the stirring bath 52, with the result that a washing agent or a chemical flows into the stirring bath 52 from the respective bottles 48 and 49.

Thus the tap water from the hydrant first passes the water filter 41 for filtering. The filtered tap water then passes the ducts, and then is branched into both ducts 46 and 47 to flow into the washing agent bottle 48 or the chemical bottle 49. The washing agent in the bottle 48 or the disinfectant in the bottle 49 is diluted with the tap water to become a solution of a predetermined concentration. This solution is therefore fed to the stirring bath 52 via the connecting duct 50 or 51.

Each of the three-way switching valves 44 and 45 has an internal fluid path which is switched from one type to another depending on electrical command signals issued by a controller 26 which works on what the current process is, i.e., a washing process, a disinfecting process, or a rinsing process. By way of practical example, for the rinsing process, the internal path of the three-way switching valve 44 is switched so as to connect the duct 42 to another circulating duct 57. Responsively to this switch, the tap water is guided to pass through the duct 57, so that the water is used for washing the outer surface and the various endoscopic ducts.

To the stirring bath 52 is connected one end of a feeding duct 53 for transferring liquid. The other end of this feeding duct 53 is linked with the circulating duct 57 via the three-way switching valve 56. Check valves 54 and 55 are inserted in the feeding duct 53.

The circulating duct 57 connects both water supply ports 16c and 17c of the washing bath 57. Between the three-way switching valve 56 and the water supply port 16c in this duct 57, a circulating pump 58 and two three-way switching valves 59 and 60 are inserted, as shown in Fig. 3.

Connected to the three-way switching valve 60 is one end of a duct 62 to lead to the endoscopic channels. The other end of this duct 62 is branched into two paths and linked with the nozzle-mounted unit 30.

When the circulating pump 58 is driven under the control of the controller 26, the washing fluid or disinfecting fluid in the stirring bath 52 passes into the circulating duct 57 via the feeding duct 53 and the three-way switching valve 56. The path of this fluid is selected into the path to the water-supply port 16c or the duct 62 for the endoscopic channels by the three-way switching valve 60 under the control of the controller 62. Thus, the outer surface and the various channels of the endoscope 20 are subjected to washing and disinfection, and the fluid which has been used for the washing and disinfection is reserved in the washing bath 5.

Another duct 61 is connected to an intermediate position of the circulating duct 57, which is between the drain port 17c and the three-way switching valve 56, and a three-way switching valve 76 and a check valve 77 are inserted in the duct 57 in this order from the drain port 17c. The three-way switching valve 76 is also linked with a drain duct 75 and subjected to control form the controller 26 for switching its internal valve paths. This switching allows the fluid in the washing bath 5 to flow out to an external outfall through the drain port 17c, three-way switching valve 76, and drain duct 75.

The three-way switching valve 59 is also connected to one end of an air-supply duct 74 in which a compressor 72 and an air filter 73 are placed. The air supplied from the compressor 72 is fed, under the control of the controller 26, to both the water-supply port 16c and the nozzle-mounted unit 30 for dehydrating water droplets and moisture on the outer surface of the washed and disinfected endoscope 20 and inside the various channels thereof.

The nozzle-mounted unit 30 is also linked with an end of a water-leakage sensing duct 63 in which there are placed a check valve 64 and a three-way switching valve 65. The remaining end of this duct 63 is linked with another compressor 66 used for sensing water leakage.

The three-way switching valve 65 is connected to one end of a duct whose other end is connected to an alcohol tank 68. Also connected to this tank 68 is one end of a duct 69, of which other end is connected to the duct 62 via a check valve 70 inserted in the duct 69.

The compressor 66 operates to supply air to the duct 63 in response to commands from the controller 26 in order to sense water leakage from the endoscope 20 or to supply the alcohol in the tank 68 to the ducts 69 and 62 in order to apply alcohol flushing to the various channels of the endoscope 20.

Furthermore, the washing bath 5 is formed to communicate with the outside via a deodorant filter 71 to remove abnormal odor in the bath 5.

Referring to Fig. 4, the nozzle-mounted unit 30 equipped with the nozzles 31, 32 and 33 for washing the endoscopic channels will now be detailed.

As shown in the figure, the nozzle-mounted unit 30 is a device which automatically connects or disconnects the nozzles 31, 32 and 33 to or from the opening of the channels formed through the insertion tube 22 of the endoscope 20. When this connecting and disconnecting operation is carried out, the endoscope 20 is already accommodated in the accommodating groove 11 on the tray 20.

More concretely, only one action makes it possible that the opening of the suction channel 22a, which opens at the suction connecting port 24a, is automatically connected with the nozzle 33 for washing the suction channel 22a mounted in the device 30 and both openings of the air-supply channel 22b (refer to Fig. 4) and water-supply duct 22c (refer to Fig. 4), which open at air/water-supply connecting ports 23a, are automatically connected with both nozzles 31 and 32 for washing the air-supply and water-supply ducts, respectively. In addition, another only one action allows those connected three nozzles 31-33 to be disconnected from the openings of the channels. For realizing those connection and disconnection actions, the nozzle-mounted unit 30 is disposed to have a predetermined positional relationship to the base-accommodating recess 12 of the tray 10.

The suction channel 22a, air-supply channel 22b, and water-supply channel 22c are endoscopic ducts which are formed to extend from the base portion 21 to the insertion tube 22 and open at the distal end surface of the insertion tube 22. The nozzle-mounted unit 30 is equipped with, as its essential components, a suction-side connecting part 40a, an air/water supply-side connecting part 40b, a pair of rail members 81a and 81b composing guide means 81, a latch type solenoid 80, a nozzle-mounted block 82, and a brush-unit mounting block 84.

The nozzle-mounted block 82 is a void box-shaped member composed of a suction-side block 82a to which the suction-side connecting part 40a is connected and an air/water supply-side block 82b to which the air/water supply-side connecting part 40b is connected. Further, the brush-unit mounting block 84 is also a void box-shaped member integrally formed with an end of the nozzle-mounted block 82, in which the end is opposite to the nozzles, as shown in Fig. 4.

The rail members 81a and 81b are fixedly disposed to be parallel to each other and positioned to provide a predetermined guide structure to the body housing 3. The nozzle-mounted block 82 is arranged slidably between the mutually parallel rail members 81a and 81b, so that this block 82 can be moved, together with the brush-unit mounting block 84, in both directions, that is, a direction advancing toward a washing-bath frame 5b and the direction opposite to the advancing direction, i.e., a direction retreating from the frame 5b.

The nozzle-mounted block 82 has sliding surfaces to be touched to the rail members 81a and 81b, a solenoid-fixing surface on which the solenoid 80 is fixed, and a nozzle-fixing surface with a step portion through which the duct-washing nozzles 31, 32 and 33 are fixedly disposed. The latch type solenoid 80 has a solenoid shaft 80a of which distal end is fixedly secured to the solenoid-fixing surface. On the back side of the nozzle-mounted block 82, plural connecting springs (not shown) are placed to couple their one ends to the back. The other ends of those connecting springs are coupled to either the rail member 81a or 81b.

The latch type solenoid 80 has a securing plate 81b, which is secured at a predetermined position of the body housing 3. In the present embodiment, the securing position of this solenoid 80 is decided such that, in cases where this endoscope washing and disinfecting apparatus 2 is in a standby for washing and disinfection, the solenoid shaft 80a is pulled in the solenoid by its magnetic force so that the nozzle-mounted block 82 is forcibly attracted to a predetermined position near to the solenoid 80.

That is, for sustaining the nozzle-mounted block 82 at the predetermined position near the solenoid 80, the magnetic force caused in the solenoid 80 is balanced with the pushing force of the elastically deformed connection springs (not shown). When the magnetic force is diminished in the solenoid, the pushing force of the connection springs moves the nozzle-mounted block 82 toward the washing-bath frame 5b.

The suction-side connecting part 40a is provided with the foregoing washing nozzle 33 for the suction channel 22a, a buffer spring 39, and a connecting L-shaped pipe 38. Meanwhile the air/water supply-side connecting part 40b is provided with the foregoing washing nozzle 31 for the air-supply channel 22b, the forgoing washing nozzle 32 for the water-supply channel 22c, an buffer spring 34, a buffer spring 35, and L-shaped pipes 36 and 37.

All the channel washing nozzles 31, 32 and 33 are fixedly secured to the nozzle-mounted block 82, respectively, and have longitudinal axes which are parallel to each other along the same plane.

Of these nozzles 31 to 33, the suction-channel washing nozzle 33 is located such that the nozzle 33 protrudes, by a predetermined length, from the front (nozzle fixing surface) of the washing bath frame 5b in front of the suction-side block 82a. Further, the air/water-supply channel washing nozzles 31 and 32 are located such that both nozzles 31 and 32 protrude, by a predetermined length, from the front (nozzle fixing surface) of the washing bath frame 5b in front of the air/water-supply side block 82b.

These nozzles 31 to 33 are placed to make their distal ends protrude in the washing bath 5 through through-holes (not shown) formed through the washing-bath frame 5b. The suction-side connecting part 40a still has a watertight sustaining member 33a, made of elastic maternal, having a cover portion and a folded portion both covering the nozzle 33, while the air/water supply-side connecting part 40b still has a watertight sustaining member 34a, made of elastic maternal, having a cover portion and a folded portion both covering both nozzles 31 and 32.

The buffer springs 39, 34 and 35 are loaded to the washing nozzles 33, 31 and 32, respectively, between the nozzle-mounted block 82 and the washing-bath frame 5b. These buffer springs 39, 34 and 35 are employed to absorb shocks caused by inserting each nozzle into each opening of each connecting port(s) 24a (23a) of the base portion 21 of the endoscope 20.

Meanwhile, with the base ends of the nozzles 31, 32 and 33, the L-shaped pipes 36, 37 and 38 are fixedly coupled. Of there nozzles, though not shown, the pipes 36 and 38 are connected with one ends of an air-supply tube and a water-supply tube on the back side of the device 30, respectively. The other ends of these tubes are connected to the duct 62 for the endoscopic channels within the body housing 3.

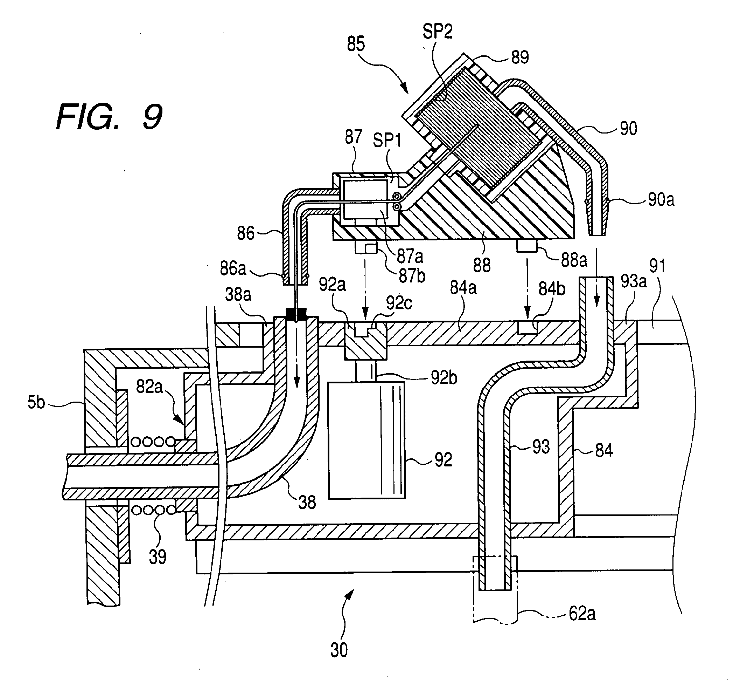

Further, the L-shaped pipe 38 is arranged to extend to the distal end of the brush-unit mounting block 84 and one end of the pipe 38 is exposed to the outside at a predetermined position of the mounting surface 84a. In the brush-unit mounting block 84, there are provided a motor 92 driven by the controller 26 in a controlled manner and a connecting L-shaped pipe 93. The L-shaped pipe 93 has a lower end, which is coupled with a fluid-supply tube 62a (refer to Fig. 9) connected with the duct 62 (refer to Fig. 3).

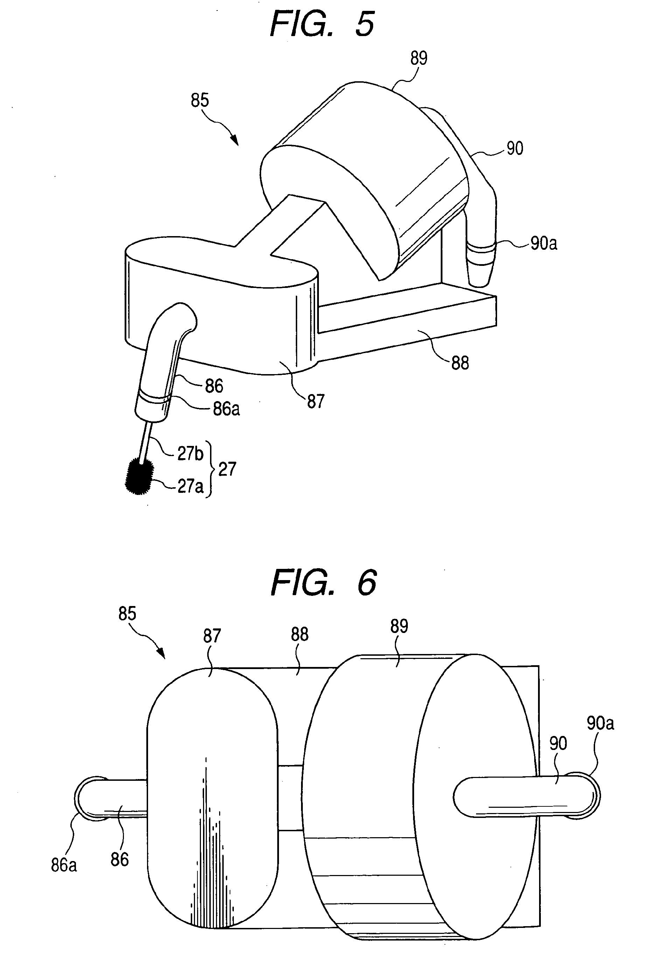

Referring to Figs. 5 to 13, the washing brush unit 85, which is detachably mounted on the brush-unit mounting unit 84, will now be described.

As shown in Figs. 5 and 6, the washing brush unit 85 is equipped with an outlet-side connecting pipe 86, a roller containing member 87, a base member 88, a brush containing member 89, and an inlet-side connecting pipe 90, in which all those members are fixed together as a one unit.

Of these, the outlet-side connecting pipe 86 has an approximate L-shaped form having both ends and one end thereof is attached at an approximately central position of the front of the roller containing member 87. The other end of the pipe 86 is directed downward. And this pipe 86 is equipped with an O-ring 86a therearound at a position near the lower end.

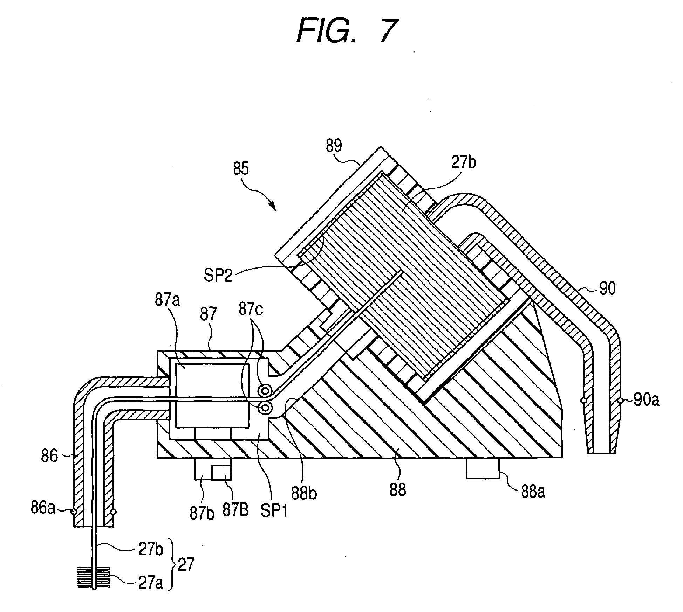

The roller containing member 87 has an outer appearance shaped into an almost elliptic cylinder (refer to Fig. 6) and an inner mid-air space SP1 which communicates with the outlet-side connecting pipe 86 (refer to Fig. 7). Inside the mid-air space SP1, a pair of rollers consisting of a driving roller 87a and a not-shown driven roller is fixedly arranged. The driving roller 87a has a shaft 87b driven to rotate beneath a base portion of the roller containing member 87. This shaft 87b passes through the base portion in an airtight manner and drives the driving roller 87a. A notch 87B is formed at a lower end of the shaft 87.

Further, on the inlet side in the mid-air space SP1 of the roller containing member 87, stick-like two brush guides 87c are disposed to extend in a width direction of the roller containing member 87. The guides 87c are formed to pinch the brush wire 27b to hold it in the upper and lower direction.

The base member 88 has a plate-like base portion and a plate-like triangular fin portion integrally formed on the base portion. Beneath the base portion, two protrusions 88a (only one is seen in Fig. 7) are formed, while inside the fin portion, a communication flow path 88b communicating with the mid-air space SP1 of the roller containing member 87 is formed, as shown in Fig. 7.

The brush containing member 89 is an approximate cylindrical member with a mid-air space SP2 therein and half-embedded in the fin portion of the base member 88 in an oblique attitude, as shown in Figs. 6 and 7. Inside the mid-air space SP2, the brush wire 27b, which is wound a not-shown bobbin and connected to the washing brush 27a, is contained. The mid-air space SP2 is connected with the communication flow path 88b extending from the mid-air space SP1 of the roller containing member 87.

The inlet-side connecting pipe 90 is bent at a mid portion thereof and one end of this pipe 90 is connected to an approximately central portion of the oblique rear side of the brush containing member 89. The other end of this pipe 90 is extended downward as shown in Fig. 7 and an O-ring 90a is provided at a position near the lower end of this pipe 90. This pipe 90 also communicates with the inner mid-air space SP2 of the brush containing member 89.

Accordingly, in this washing brush unit 85, both connecting pipes 90 and 86 serves as members providing input and output ports, so that the inlet-side connecting pile 89, the inner mid-air space SP2 of the brush containing member 89, the communication flow path 88b of the base member 88, the inner mid-air space SP1 of the roller containing member 87, and the outlet-side connecting pipe 86 are communicated with each other.

The brush device 27 is thus installed in those mutually communicated spaces of the washing brush unit 85 such that the wound brush wire 27b, which resides in the space SP2, extends through the communication flow path 88b, pressed between the rollers (one of which is the driving roller 87c) in the space SP1, and the outlet-side connecting pipe 86. And the brush 27a can be pushed forward from the lower end of the pipe 86 and pulled back into the pipe 86.

It is possible that that washing brush unit 85 is detachably mounted on the brush-unit mounting block 84, which is disposed to the outside.

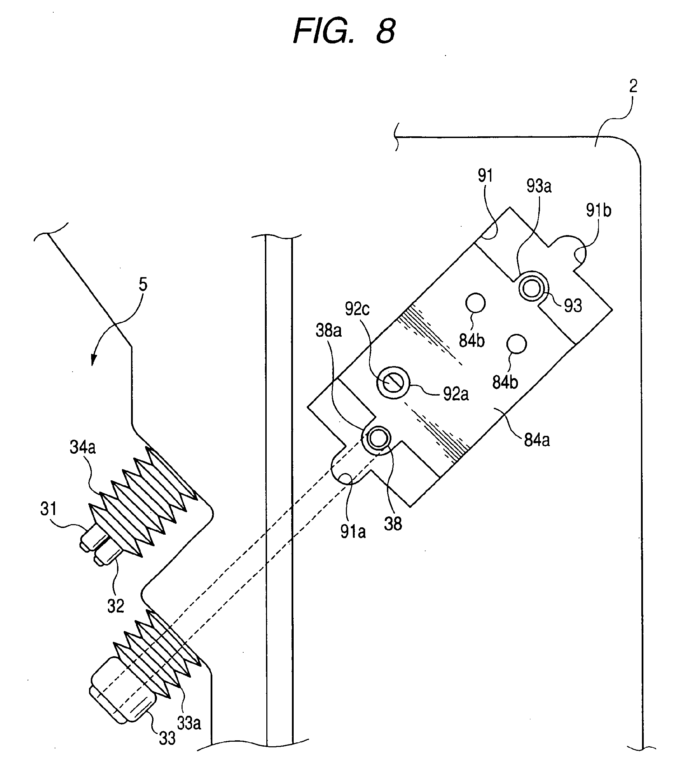

This mounting structure will now be detailed. As shown in Figs. 8 and 9, there is formed an approximately rectangular sliding groove 91 on the nozzle-mounted unit 30. In both longitudinal portions of this sliding groove 91, there are formed semicircular engaging recesses 91a and 91b. In addition, this sliding groove 91 allows a mounting surface 84a of the brush-unit mounting block 84 to be disposed to the outside.

This mounting surface 84a is disposed to the outside in a state where this surface has the same level as the edge surface of the body housing 3. In the brush-unit mourning block 84, an L-shaped pipe 38 is embedded so that its one end reaches a front-side predetermined position in the mounting surface 84a and the connecting L-shaped pipe 93 is embedded so that its one end reaches a rear-side predetermined position in the mounting surface 84a. The openings of those pipes 38 and 93 in the mounting surface 84a are surrounded by semicircular engaging protrusions 38a and 93a, respectively. As shown in Fig. 9, the brush-unit mourning block 84 contains a motor 92 having a shaft 92b coupled with one end of a connector 92a. The other end of this connector 92a is also exposed to the outside in the mounting surface 84a, as shown in Figs. 8 and 9. This connector 92a is mounted through the upper plate providing the mounting surface 84a and is freely rotatable there in an airtight manner, thanks to a not-shown sealing member. In the mounting surface 84a, there are also formed two engaging recesses 84b, as shown in Figs. 8 and 9.

Further, the unit mounting surface 84a is shorter in longitudinal length than the sliding groove 91, so that the nozzle-mounted unit 30 can be slide-moved freely with no interruption when this device 30 is attached to and released from the body housing 3 to connect or disconnect the nozzles 31-33 to and from the connecting ports 23a and 24a of the endoscope 20.

The washing brush unit 85 is mounted onto the unit mounting space 84a such that, as shown in Fig. 9, the connecting pipes 86 and 90 are inserted into the openings of the pipes 38 and 93 in the surface 84a, respectively, the shaft 87b of the driving roller 87a is engaged with the connector 92 of the motor 92 in the surface 84a, and the two protrusions 88a are engaged with the two engaging recesses 84b, respectively. The connections between the pipes 86 and 38 and between the pipes 90 and 93 are realized air-tightly by the O-rings 86a and 90a, respectively.

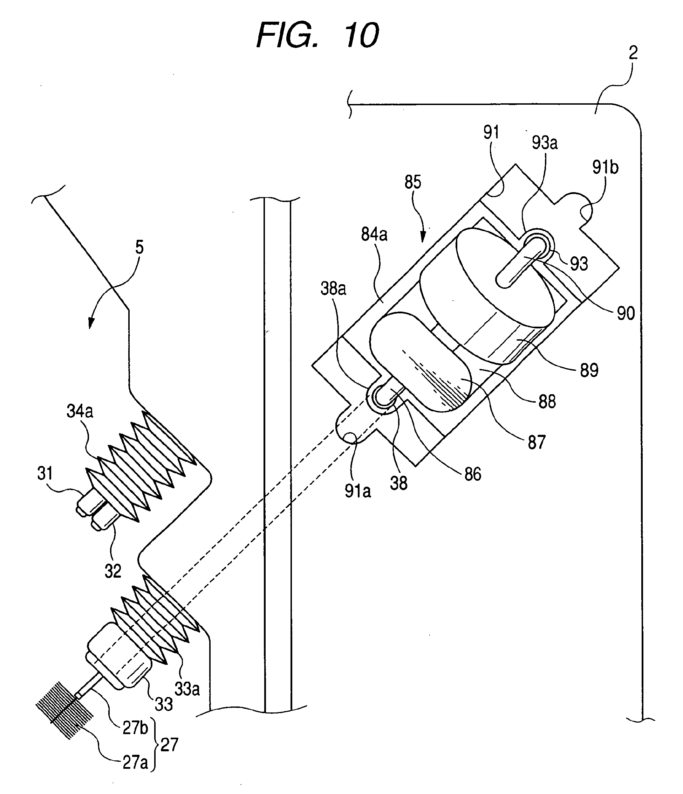

Thus, as illustrated in Fig. 10, an operator can mount the washing brush unit 85 on the unit mounting surface 84a, that is, the nozzle-mounted unit 30 which is located at the upper corner of the body housing 3 of the apparatus 2.

When the above mounting action is done, the shaft 87b of the driving roller 87a is inserted in a recess 92c of the connector 92a. This recess 92c is formed to be coupled with a notch formed on the end of the shaft 87b, so that the rotational force of the motor 92 is transmitted to the driving roller 87a via such a coupling.

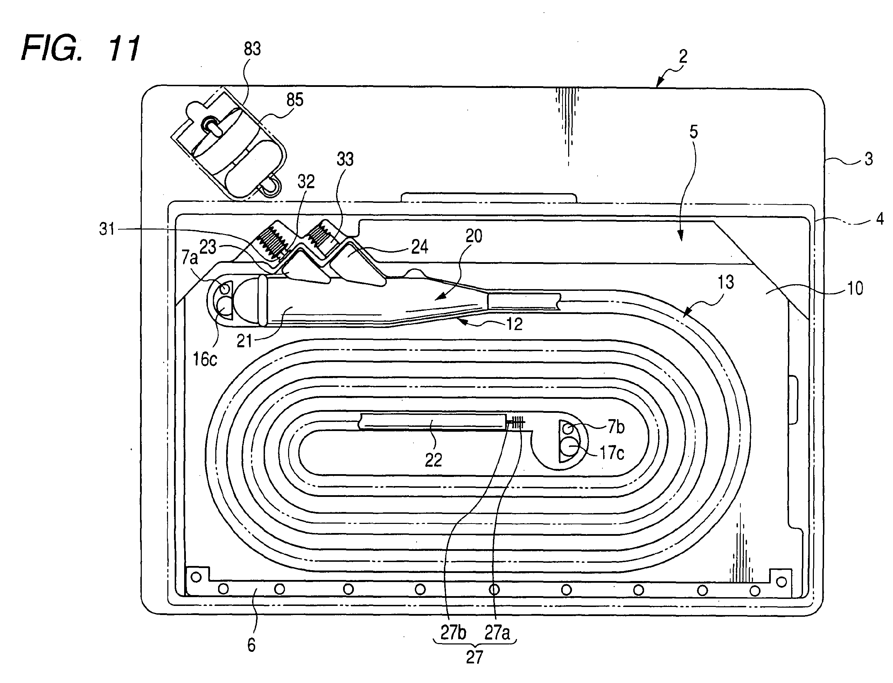

In the present endoscope washing and disinfecting apparatus 2, a used endoscope 20 is accommodated in the accommodating groove 11 (i.e., recess 12 and groove 13) on the tray 10, this tray 10 is installed in the washing bath 5 of the body housing 3, and the top cover 4 is closed, as shown in Fig. 11.

In addition, the washing brush unit 85 is mounted at the predetermined upper corner position, i.e., at the mounting region 84a on the upper surface of the body housing 3, and then the cover 83 is closed. In response to an operator's push action at a start switch on the operation panel 8, predetermined processing for washing, rinsing, disinfecting, re-rinsing, and dehydrating processes for the endoscope 20 is activated under the control of the controller 26 working on not-shown software programs preset in the controller 26 in advance. Therefore, such processes are executed in sequence.

To be specific, when the start switch is pushed, the nozzle-mounted unit 30 is moved forward by a drive force generated by the solenoid 80, resulting in that the nozzles 31 and 32 are coupled with the openings of the air/water-supply connecting ports 23a and, concurrently with this, the nozzle 33 is coupled the opening of the suction connecting port 24a.

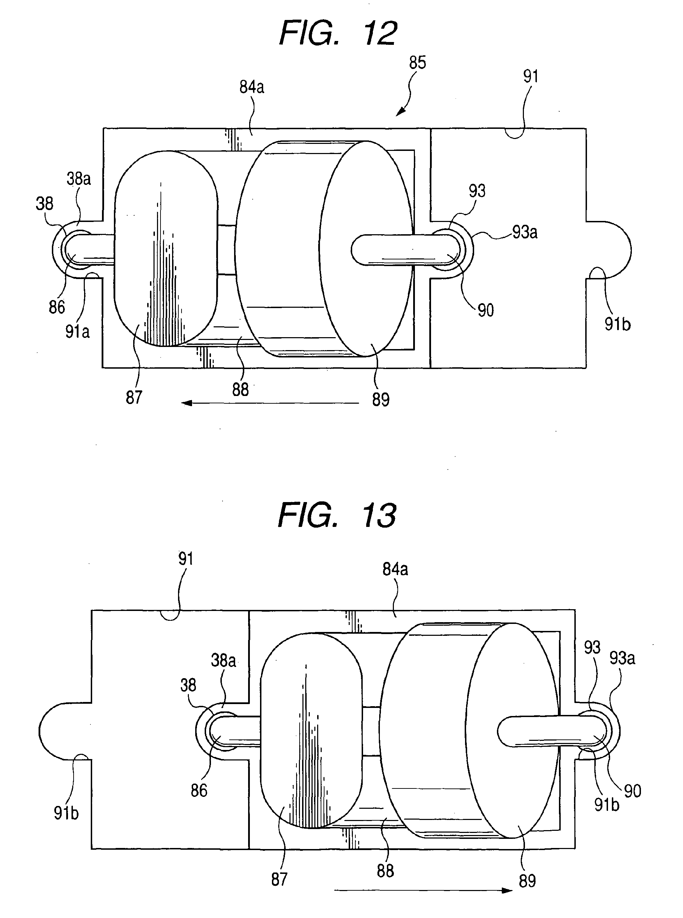

This forward movement of the nozzle-mounted unit 30 accompanies the washing brush unit 85 mounted on the mounting surface 84a of the unit 30. As shown in Fig. 12, on completion of the movements of both units 30 and 85, the engaging protrusion 38a of the mounting surface 84a is engaged with the engaging recess 91a of the sliding groove 91.

After this, the endoscope washing and disinfecting apparatus 2 performs a washing press with the endoscope 20. In the washing process, the circulating pump 48 is driven to circulate the washing fluid to the water supply port 16c through the circulating duct 57 and then flow through the mounting groove 11 on the tray 10. Hence the outer surface of the endoscope 20 is washed. In parallel with this washing, the generated washing fluid also flows from the circulating duct 57 to the branched duct 62, and is fed to the nozzles 31, 32 and 33 of the nozzle-mounted unit 30. Hence each of the nozzles 31, 32 and 33 feeds the washing fluid to each of their assigned ducts 22b, 22c and 22a for washing the inside thereof.

Referring to Fig. 9, how the washing fluid flows in the nozzle-mounted unit 30 will now be detailed. The washing fluid that flows through the duct 62 is shunted to the connecting L-shaped pipe 93 via the fluid-supply tube 62a, and then fed to the washing brush unit 95. The washing fluid passes in sequence the inlet-side connecting pipe 90, inner-mid space SP2 in the brush containing member 89, communication flow path 88b of the base member, inner-mid space SP1 of the roller containing member 87, and outlet-side connecting pipe 86. Then the washing fluid is supplied into the suction duct 22a of the endoscope 2 via the connecting L-shaped pipe 38.

In parallel with this flow, under the control of the controller 26, the electric motor 92 is driven to provide the driving roller 87a so that the roller 87a revolves in the clockwise and counterclockwise directions in a controlled manner. Hence the brush wire 27b, which is pressed between the two rollers 87a, is obliged to advance and retreat repeated. The brush 27a is therefore extended from the nozzle 33 to be inserted in the suction channel 22a and is made to repeatedly advanced and retreat (i.e., inserted and pulled back) therealong.

With the help of the washing fluid that passes through the suction channel 22a, those repeated advancing and retreating actions of the brush 27a results in brushed washing to the inside wall of the suction channel 22a. In this washing process, the circulating pump 58 is in operation without rest to constantly supply the washing fluid to the suction channel 22a.

On completion of the washing process, the controller 26 commands to the motor 90 to stop when the brush 27a returns to its initial position determined in the path of the outlet-side connecting pipe 86 which is part of the washing brush unit 85. This washing process is then followed by a rinsing process.

In this way, both the outer surface and the suction channel (duct) 22a of the endoscope 20 are washed. The rinsing process and succeeding disinfecting and a second rinsing process are carried out similarly to the foregoing, whereby the endoscope 20 is washed and disinfected.

When the series of processes for washing/disinfecting the endoscope 20 is finished, the operator picks up the tray 10 with the endoscope still accommodated thereon, before ending the washing and disinfecting processes. Meanwhile, when the washing brush unit 85 is desired to be replaced by another new one, the used washing brush unit 85 can easily be detached from the mounting surface 84a of the body housing 3 by picking it up. The detached unit 85 can be re-used or disposed of.

As described, in the present endoscope washing and disinfecting apparatus 2, the device 27 having the long wire 27b and the brush 27a, which can be used effectively for brush-washing the suction channel 22a of the endoscope 2, is accommodated in the washing brush unit 85. This unit 85 is also detachable to the apparatus 2 in an easy and reliable manner. For washing the suction channel 22a, the brush 27a is driven to advance and retreat through the suction channel 22a in an automatic fashion, without manual washing work. In addition, when the brush device 27 is used up, it is sufficient to dispose of the whole washing brush unit 85 as medical waste. The long brush device 27 is already wound in the unit 85 when being detached from the housing 3, so that there is no need to fold back the long brush device 27, whereby the waste disposal can be done easily.

As a result, the endoscope washing and disinfecting apparatus 2 can be used to wash and disinfect used endoscopes, in particular, to the suction channel of each endoscope to recover the sanitary conditions of the endoscopes with reliability and effectiveness in washing work. Thus, the manual troublesome washing/disinfecting work can be lessened.

### (Second embodiment)

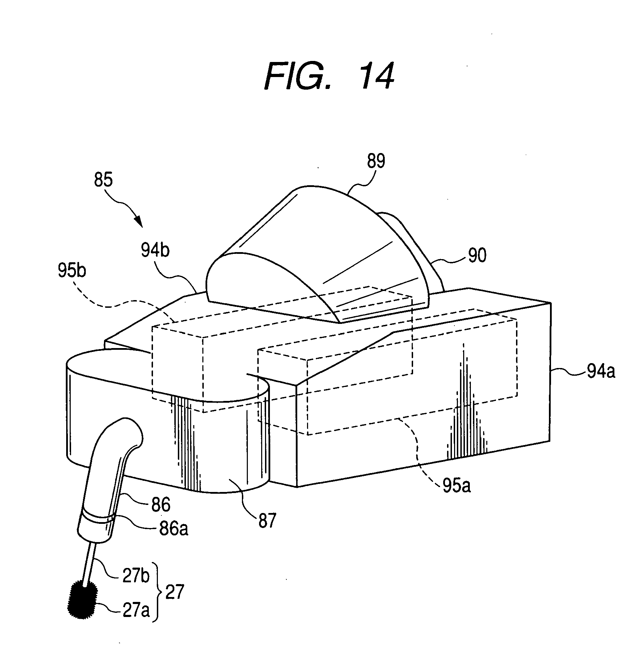

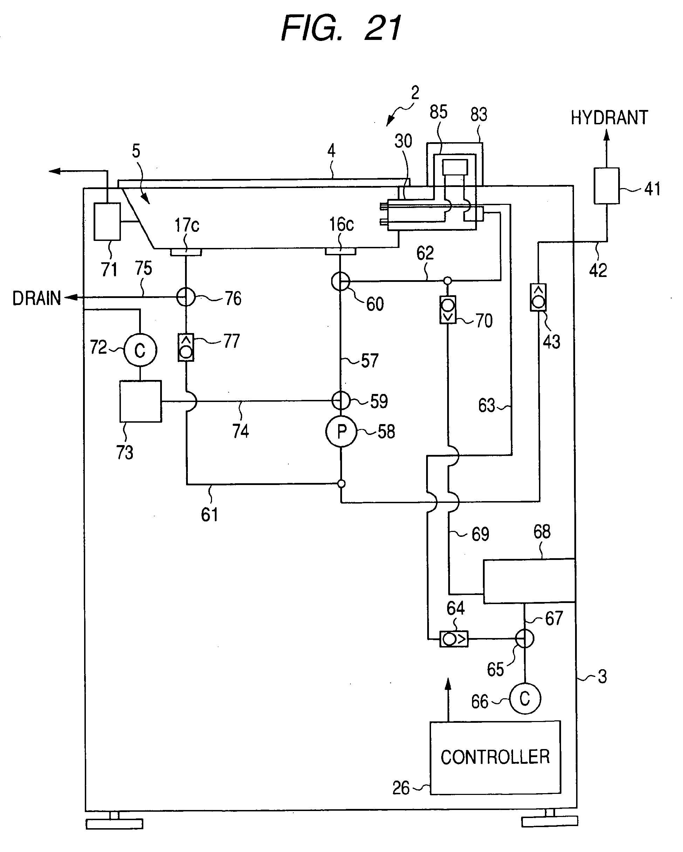

Referring to Figs. 14 - 21, a second embodiment of the medical instrument washing and disinfecting apparatus according to the present invention will now be described. In the present embodiment, the identical or similar components to those in the first embodiment will be given the same reference numerals for the sake of a simplified of omitted explanation.

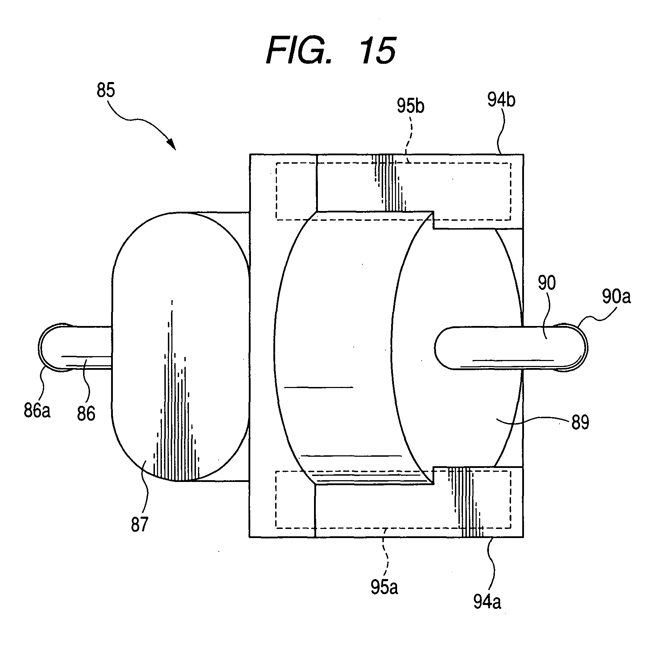

As shown in Figs. 14 and 15, on both sides of the brush containing member 89, a washing brush unit 85 according to the present embodiment is equipped with two tank 94a and 94b for containing the washing fluid and the disinfecting fluid which are liquid concentrate. Both tanks 94a and 94b are located on the rear side of the roller containing member 87.

Of both tanks 94a and 94b, the tank 94a, located on the left when viewed along a direction from the inlet side to the outlet side, contains the washing fluid, while the tank 94b on the opposite side contains the disinfecting fluid.

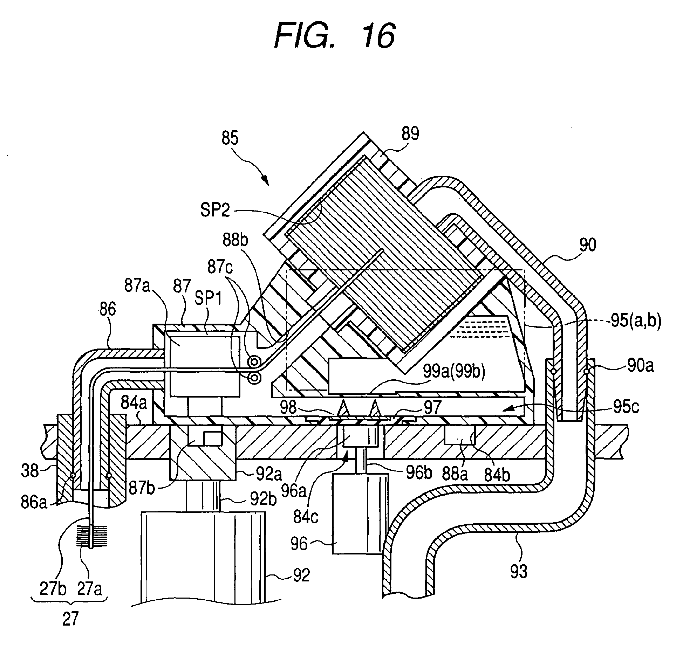

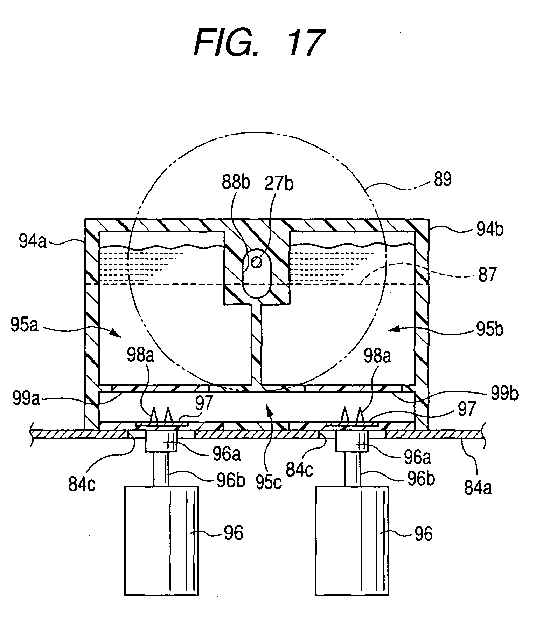

The washing fluid tank 94a comprises a housing equipped with a reservoir 95a in which the washing fluid is contained. The remaining disinfecting fluid tank 94b comprises a housing equipped with a reservoir 95b in which the disinfecting fluid is contained. As shown in Figs. 16 and 17, each of the reservoirs 95a and 95b has a base formed by a resin membrane 99a (99b) made of polyester.

The base of each tank 94a (94b) directly faces each elastic-made plate 97 formed on the base of the washing brush unit 85. On each plate 97, plural prong members 98 are disposed so as to directly stand up. Between the base of the washing brush unit 85 and the reservoirs 95a and 95b, there is formed a flow-out path 95c communicating with the inner mid-air space SP1 of the roller containing member 87. Hence the prong members 98 are present in the flow-out path 95c.

Like the first embodiment, the washing brush unit 85 is mounted on the mounting surface 84a of the brush-unit mounting block 84 (refer to Fig. 16). The upper plate of the unit 84, which provides the mounting surface 84a, has two through-holes 84c each facing each of the plates 97 without a gap when the unit 85 is mounted.

In the through-holes 84c, cylinders 96a are placed respectively. Each cylinder 96a is linked with a shaft 96b extended from a solenoid 96, which is installed in the brush-unit mounting block 84. The two solenoids 96 are individually driven by control signals from the controller 26.

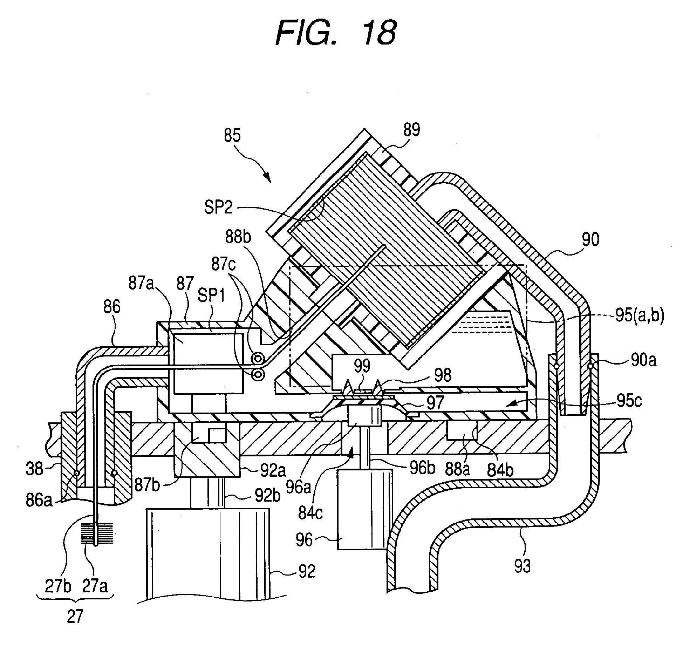

Hence, in the present endoscope washing and disinfecting apparatus 2, when the washing or disinfecting process is started, either one of the solenoids 96, which corresponds to a specified one of the reservoirs 95a and 95b, is selectively driven in response to a control signal from the controller 26. This drive forces the corresponding cylinder 96a to raise upward the plate 97 of the base of the mounted unit 95. The plate 97 is obliged to elastically be deformed so that the prong members 98 are also raised. The raised prong members 98 forcibly picks and breaks the thin membrane 99 (base) of the reservoir 95a for the washing fluid or the reservoir 95b for the disinfecting fluid (refer to Figs. 16 and 18).

That is, when the washing process is started, the controller 26 operates to control the drive of each solenoid 96 assigned to the washing side, with the result that the prong members 98 picks and breaks the thin membrane 99 of the washing-fluid reservoir 95a. The same is true of the disinfecting process which requires the disinfecting-fluid reservoir 95b. After such a drive, the controller 26 operates to return the solenoid 96 to its initial state.

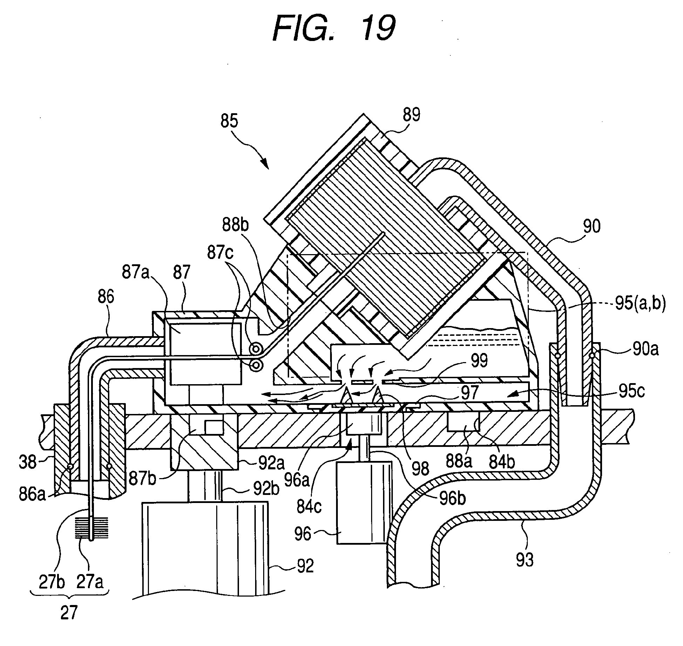

Thus, as shown in Fig. 19, the washing fluid or the disinfecting fluid contained each reservoir 95a (95b) is flowed out from the picked and broken portions of each reservoir 95a (95b). In parallel with this picking/breaking operation, the controller 26 operates to drive the circulating pump 58 to supply the tap water to the washing brush unit 85, whereby the washing fluid or the disinfecting fluid is diluted with the water in the inner space SP1 of the roller containing member 87. The diluted washing or disinfecting fluid, which can be used for the actual washing or disinfection, is fed to the suction channel 22a via the pipes 86 and 38 and nozzle 33, and then flowed into the washing bath 5.

Then the diluted washing or disinfecting fluid is then circulated though various ducts in the housing 3 so the fluid is stirred to a predetermined concentration. As stated in the first embodiment, during the washing process, the brush device 27 is driven to apply brushed washing to the suction channel 22a of the endoscope 20. Incidentally, the brush device 27 may be driven in the other processes, such as disinfecting process, other then the washing process.

In this way, the washing brush unit 85 can be formed to have the tanks 94 containing the washing and disinfecting fluid, respectively, thereby eliminating the labor to load the housing 3 with the washing agent bottle 85 and the chemical bottle 49, unlike the first embodiment.

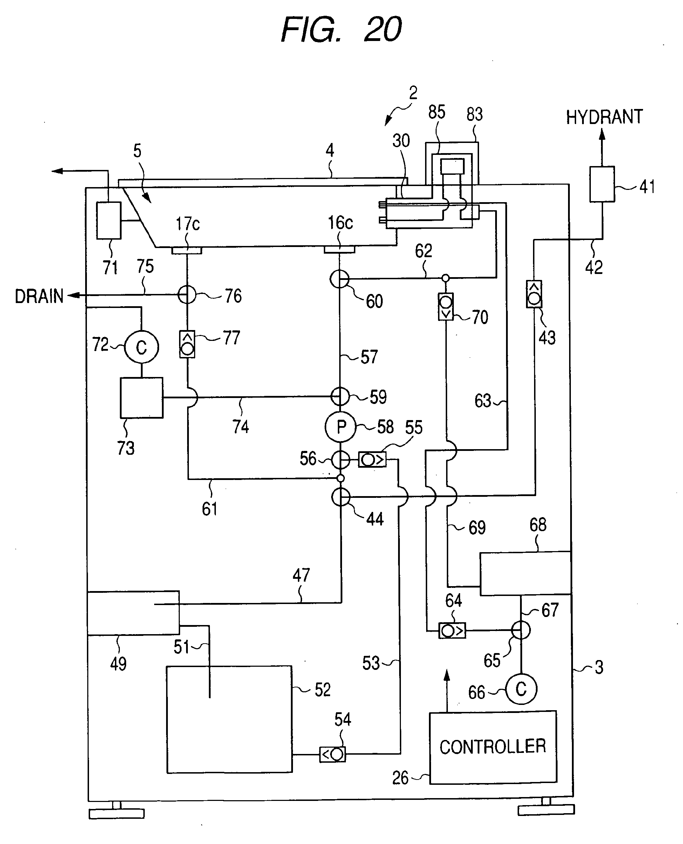

Of course the washing brush unit 85 may have only the washing fluid tank 94a. If the apparatus 2 is designed so, it is no longer necessary to install the washing agent bottle 48, duct 46, and three-way switching valve 45 (refer to Fig. 3) in the apparatus 2, as shown in Fig. 20.

Alternatively, the washing brush unit 85 is provided with both tanks 94a and 94b for the washing and disinfecting fluid, respectively, whereby as shown in 21, the piping structure of the apparatus 2 can be simplified. Specifically, the three three-way switching valves 44, 45 and 56, two check valves 54 and 55, five ducts 46, 47, 50, 51 and 53, two bottles 48 and 49, and stirring bath 52 (refer to Fig.. 3) are not required.

As a result, the endoscope washing and disinfecting apparatus 2 is able to enjoy, in addition to the similar advantages to those gained in the first embodiment, a simplified piping structure and less-space housing structure, which make the apparatus 2 more compact.

Although the description above contains many specificities, these should not be construed as limiting the scope of the invention but as merely providing illustrations of some of the presently preferred embodiments of the present invention. Thus the scope of the present invention should be determined by the appended claims.

## Claims

1. An apparatus for washing and disinfecting a medical instrument with a duct, comprising:
an accommodating member on which the medical instrument is accommodated;
a bath member providing a washing bath in which the accommodating member is placed;
a washing unit washing at least the duct of the medical instrument accommodated on the accommodating member and placed in the washing bath; and
a brushing unit detachably mounted on the bath member and formed to have a brush brushing an inside of the duct when the endoscope is washed and disinfected by the washing unit.

2. The apparatus of claim 1, wherein the medical instrument is an endoscope with an insertion tube having the duct therein and being inserted into an object being examined.

3. The apparatus of claim 1, wherein
the washing unit is equipped with a first washing/disinfecting unit washing and disinfecting an outer surface of the medical Instrument and a second washing/disinfecting unit washing and disinfecting the inside of the duct of the medical instrument, and
the brushing unit is configured to enable the brush to advance and retreat along inside the duct when the second washing/disinfecting unit washes the inside of the duct.

4. The apparatus of claim 3, wherein
the accommodating member a groove in which the medical instrument is accommodated in a predetermined form thereof and
the first washing/disinfecting unit comprises first fluid supplying means for supplying along the groove washing fluid, disinfecting fluid, and rinsing water in a state where the endoscope is accommodated in the groove.

5. The apparatus of claim 4, wherein the second washing/disinfecting unit comprises a coupling member detachably coupling a nozzle to an opening of the duct of the medical instrument and second fluid supplying means for supplying at least washing fluid, disinfecting fluid, and rinsing water to the duct through the nozzle; and
the apparatus further comprising
a driver driving the brush to advance and retreat along inside the duct and
a controller controlling the driver such that the brush is inserted into the duct through the nozzle and is made to advance and retreat over an entire longitudinal path of the duct, in cases where the second fluid supplying means supply at least the washing fluid.

6. The apparatus of claim 5, wherein
the duct of the medical instrument consists of a plurality of ducts each having an opening,
the nozzle consists of a plurality of nozzles each detachably coupled to the opening of each of the plural ducts, and
the brushing unit is configured to allow the brush to be inserted into a given duct of the plural ducts.

7. The apparatus of claim 6, wherein
the medical instrument is an endoscope,
the plural ducts include an air-supply duct, a water-supply duct and a suction duct, and
the given duct is assigned to the suction duct.

8. The apparatus of claim 5, wherein the base member contains a fluid source supplying at least the washing fluid, the disinfecting fluid, and the rinsing water to the first and second fluid supplying means.

9. The apparatus of claim 8, wherein
the fluid source comprises a tank in which the washing fluid and the disinfecting fluid are stored as liquid concentrate and diluting means for diluting the liquid concentrate in the tank and supplies the diluted liquid to the first and second fluid supplying means.

10. The apparatus of claim 9, wherein
the brushing unit comprises a mechanism for detachably loading the brushing unit to the coupling member and an inner fluid path establishing communication between an inflow port to which the diluted liquid flows in and an outflow port from which the diluted liquid flowing into the inflow port flows out,
wherein the outflow port is connected to the nozzle of the coupling member via an outflow-side fluid path.

11. The apparatus of claim 10, wherein
the brushing unit comprises
a wire wound unwindably and disposed in the inner fluid path and formed to have a distal end to which the brush is loaded and
a roller disposed in the inner fluid path and equipped a roller enabling the wire to advance and retreat along an extending direction thereof depending on a drive force coming from the driver.

12. The apparatus of claim 11, wherein
the brushing unit is configured to such that, when the brushing unit is mounted on the coupling member, a rotary shaft of the roller is positioned at a shaft transmitting the drive force from the driver and coupled with the shaft to force the roller, the inflow port is positioned at an inflow-side flow path communicating with the diluting means and coupled with the inflow-side flow path, and the outflow port is positioned at the outflow-side flow path so as to communicate thereof.

13. The apparatus of claim 12, wherein
the brushing unit comprises a washing/disinfecting unit washing and disinfecting an inside of the duct of the medical instrument and
the brushing unit is configured to enable the brush to advance and retreat along inside the duct when the washing/disinfecting unit washes the inside of the duct.

14. The apparatus of claim 13, wherein the washing/disinfecting unit comprises a coupling member detachably coupling a nozzle to an opening of the duct of the medical instrument and fluid supplying means for supplying at least washing fluid, disinfecting fluid, and rinsing water to the duct through the nozzle; and
the apparatus further comprising
a driver driving the brush to advance and retreat along inside the duct and
a controller controlling the driver such that the brush is inserted into the duct through the nozzle and is made to advance and retreat over an entire longitudinal path of the duct, in cases where the fluid supplying means supply at least the washing fluid.

15. The apparatus of claim 5, wherein
the brushing unit comprises
a fluid path providing a communication between an inflow port to which the water flows and an outflow port from which the water that has passed through the fluid path flows out;
a tank storing at least one of the washing fluid and the disinfecting fluid;
a mechanism automatically allowing the fluid in the tank to flow out when the brushing unit is mounted on the coupling member; and
a guiding path guiding the fluid that has flowed out from the tank to the fluid path.

16. The apparatus of claim 15, wherein
the mechanism comprises picking/cracking means mounted on the coupling member and formed to pick and crack and the tank in response to a control signal and control means for providing the control signal.

17. The apparatus of claim 16, wherein
the tank includes two tanks respectively storing therein the washing fluid and the disinfecting fluid and
the picking/cracking means are arranged tank by tank for the two tanks, respectively.

18. The apparatus of claim 16, wherein
the tank is partly made of a thin membrane and
the picking/cracking means comprises a prong member and a drive source for driving the prong member so as to move toward the thin membrane of the tank in response to the control signal.

19. The apparatus of claim 16, wherein the tank contains liquid concentrate of at least one of the washing fluid and the disinfecting fluid,
the apparatus further comprising diluting means for diluting the liquid concentrate flowing out from the tank by making water flow into the fluid path.

20. A detachable type of brushing apparatus, comprising:
a detachable member detachably mounted to a detachable portion of a washing and disinfecting apparatus for washing and disinfecting a medical instrument with a duct and formed to providing an approach path to the duct of the medical instrument;
a flow path communicating with the approach path; and
a brushing unit disposed in the flow path and formed to have a brush advancing an inside of the duct through the approach path and brushing the inside of the duct, when the duct is subjected to at least washing.

21. The detachable type of brushing apparatus of claim 20, further comprising
a tank containing at least one of washing fluid and disinfecting fluid necessary for washing and disinfection of the duct and
a guiding path guiding the fluid contained in the tank to the flow path.

22. The detachable type of brushing apparatus of claim 21,
wherein the brushing unit comprises a winding type of wire having an end to which the brush is loaded and being allowing the brush to advance and retreat between the fluid path and the duct through the approach path depending on a drive force.

23. The detachable type of brushing apparatus of claim 21, comprising a mechanism automatically allowing the fluid in the tank to flow out into the guiding path when the detachable member of the brushing unit is mounted on the detachable portion of the washing and disinfecting apparatus.

24. The detachable type of brushing apparatus of claim 23,
the mechanism comprises picking/cracking means mounted to the washing and disinfecting apparatus and formed to pick and crack the tank in response to a control signal and control means for providing the control signal.

25. The detachable type of brushing apparatus of claim 24, wherein
the tank includes two tanks respectively storing therein the washing fluid and the disinfecting fluid and
the picking/cracking means are arranged tank by tank for the two tanks, respectively.

26. The detachable type of brushing apparatus of claim 24, wherein
the tank is partly made of a thin membrane and
the picking/cracking means comprises a prong member disposed to be opposed to the thin membrane of the tank and formed to be moved toward the thin membrane by a force from a drive source in response to the control signal.

27. The detachable type of brushing apparatus of claim 26, comprising a partition member watertightly portioning the guiding path inside, locating to be opposed to the thin membrane of the tank, and having an elastic portion located with the drive source rearward.

28. The detachable type of brushing apparatus of claim 24, wherein the tank contains liquid concentrate of at least one of the washing fluid and the disinfecting fluid,
the fluid path is formed to have a space in which the liquid concentrate flowing out from the tank is diluted with water supplied to the fluid path.
